# EUROPEAN PATENT APPLICATION

(11) **EP 4 840 505 A1**
(43) Date of publication of application: **18.11.2026**
(21) Application number: 26178199.1
(22) Date of filing: 12.05.2026
(51) Int. Cl.: A61N 5/10

(54) **RADIATION THERAPY CONTROL METHOD, ELECTRONIC DEVICE, STORAGE MEDIUM, AND PROGRAM PRODUCT**

(30) Priority: 16.05.2025 CN 202510637515
(71) Applicant: Our United Corporation, Xi'an, Shaanxi 710018 (CN)
(72) Inventor: LIU, Haifeng, Shaanxi, 710018 (CN); ZHU, Zhaohui, Shaanxi, 710018 (CN); HAN, Jun, Shaanxi, 710018 (CN)
(74) Representative: Murgitroyd & Company

(57) **Abstract**

The present disclosure relates to the technical field of medical treatment, and in particular, to a radiation therapy control method, an electronic device, a storage medium, and a program product. The method includes: acquiring a fluoroscopy image of a patient taken at a current moment during a radiation therapy process; and controlling the radiation therapy process for the patient through at least one intelligent agent based on the fluoroscopy image and a target image taken for the patient before radiation therapy.

## Description

### TECHNICAL FIELD

The present disclosure relates to the technical field of medical treatment, and in particular, to a radiation therapy control method, an electronic device, a storage medium, and a program product.

### BACKGROUND

Real-time monitoring and adjustment are key to dynamic radiation therapy. At present, there is a problem of insufficient response to sudden deviations beyond a preset rule range when dealing with complex dynamic treatments in the related technologies, and it is difficult to compensate for the deviation dose in a timely manner. These insufficient things affect the safety and accuracy of the dynamic radiation therapy.

### SUMMARY

In a first aspect, a radiation therapy control method is provided, the method includes: acquiring a fluoroscopy image of a patient taken at a current moment during a radiation therapy process; and controlling the radiation therapy process for the patient through at least one intelligent agent based on the fluoroscopy image and a target image taken for the patient before radiation therapy.

In a second aspect, a radiation therapy control apparatus is further provided. The radiation therapy control apparatus includes an acquisition module and a processing module. The acquisition module is configured acquire a fluoroscopy image of a patient taken at a current moment during a radiation therapy process; the processing module is configured to control the radiation therapy process for the patient through at least one intelligent agent based on the fluoroscopy image and a target image taken for the patient before radiation therapy.

In a third aspect, an electronic device is also provided, which includes a processor and a memory configured to store instructions executable for the processor; where the processor is configured to execute the instructions to implement any one of the radiation therapy control methods in the above-mentioned first aspect.

In a fourth aspect, a computer-readable storage medium is provided, and the computer-readable storage medium stores instructions. When the instructions in the computer-readable storage medium are executed by a device, the device is capable of performing any one of the radiation therapy control methods in the above-mentioned first aspect.

In a fifth aspect, a computer program product is provided, and the computer program product includes computer instructions that, when executed on a processor of a device, enable the device to perform any one of the radiation therapy control methods in the above-mentioned first aspect.

### BRIEF DESCRIPTION OF THE DRAWINGS

In order to illustrate technical solutions in the embodiments of the present disclosure more clearly, the accompanying drawings needed to be used in descriptions for the embodiments will be introduced briefly. Obviously, the accompanying drawings in the description below are merely a portion of accompanying drawings of some embodiments of the present disclosure, and for a person of ordinary skill in the art may obtain other accompanying drawings according to these accompanying drawings without paying any creative effort.
FIG. 1 is a schematic diagram of structures of a radiation therapy control system provided in the embodiments of the present disclosure;
FIG. 2 is a flowchart of a radiation therapy control method provided in the embodiments of the present disclosure;
FIG. 3 is a flowchart of another radiation therapy control method provided in the embodiments of the present disclosure;
FIG. 4 is a flowchart of yet another radiation therapy control method provided in the embodiments of the present disclosure;
FIG. 5 is a flowchart of yet another radiation therapy control method provided in the embodiments of the present disclosure;
FIG. 6 is a schematic diagram of structures of a radiation therapy control apparatus provided in the embodiments of the present disclosure; and
FIG. 7 is a schematic diagram of structures of an electronic device provided in the embodiments of the present disclosure.

### DETAILED DESCRIPTION

In the embodiments of the present disclosure, the terms "first", "second", etc., are only used for descriptive purposes and cannot be understood as indicating or implying relative importance or implying the number of technical features indicated. Thus, features limited with the wordings such as "first" or "second" may explicitly or implicitly include one or more of the features.

In the embodiments of the present disclosure, the terms "include/comprise", "contain", or any other variation thereof are intended to encompass non-exclusive inclusion, such that a process, method, article, or apparatus including a series of elements includes not only those elements, but also other elements not explicitly listed, or elements inherent to such process, method, article, or apparatus. Without further limitations, elements limited by the statement 'including one...' does not exclude the existence of other identical elements in the process, method, article, or device that includes that element.

"A and/or B" includes the following three combinations: only A, only B, and a combination of A and B.

During dynamic radiation therapy process, many factors such as a patient position, a shape and a size of a tumor, and a density of surrounding normal tissues may undergo instantaneous changes, resulting in various unpredictable deviations. However, most existing monitoring and adjustment systems operate based on preset rules and models. When encountering unexpected situations beyond the preset scope, it is difficult to make accurate judgments and take effective response measures quickly. Often, it takes some time to re-evaluate and adjust strategies, which leads to delays in the entire treatment process at critical moments and greatly affects the treatment progress. On the other hand, it is difficult to achieve timely and effective compensation for the dose issues caused by these deviations. The accurate administering of dose is one of the key factors for the success of dynamic radiation therapy. Once there is a deviation, the originally planned dose distribution will be broken, and normal tissues may be overexposed, while a dose at a tumor site may be insufficient, seriously affecting the treatment effect. Although with the existing technology, dose issues may be detected after detecting deviations, it is difficult to accurately calculate the dose value that needs to be compensated and determine the optimal compensation method in a short period of time. This leads to the inability to correct dose deviations in a timely manner, posing a serious threat to the safety and accuracy of patient treatment.

Based on the above technical problems, a radiation therapy control method is provided in the embodiments of the present disclosure, which involves acquiring a fluoroscopy image of a patient taken at a current moment during a radiation therapy process; and controlling the radiation therapy process for the patient through at least one intelligent agent based on the fluoroscopy image and a target image taken for the patient before radiation therapy. In this method, with acquiring changes of the target area of the patient, capabilities of information processing and decision-making of the intelligent agent are used to dynamically regulate the radiation therapy process, improving the accuracy and safety of radiation therapy, thereby assisting in achieving precise targeting of the target area and enhancing the overall effect of radiation therapy.

The radiation therapy control method, an electronic device, a storage medium, and a program product provided in the present disclosure is introduced below.

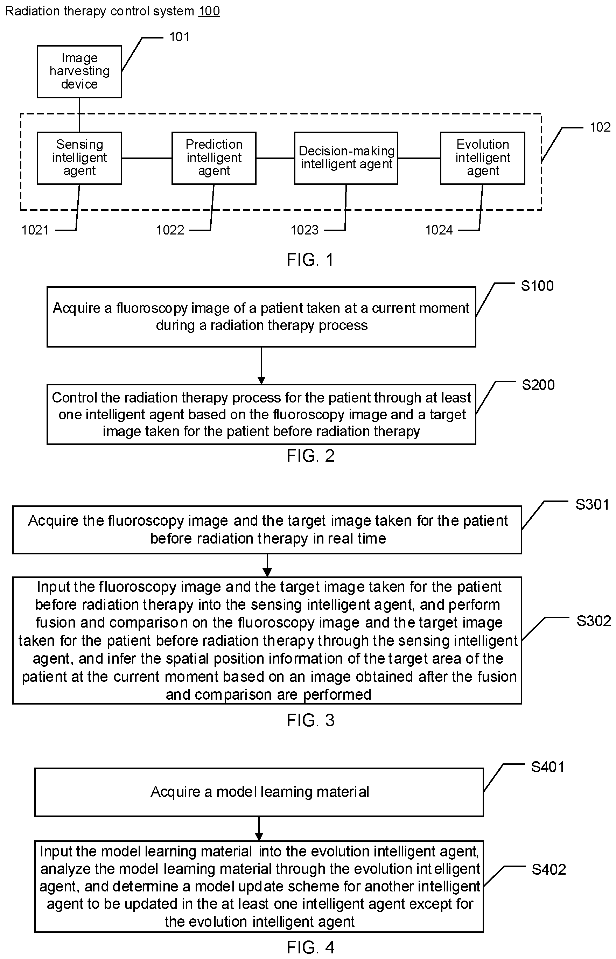

In some embodiments, a radiation therapy control system is provided in the present disclosure, as shown in FIG. 1, the radiation therapy control system 100 includes an image harvesting device 101 and an intelligent agent 102.

In some embodiments, the image harvesting device 101 is used to take a fluoroscopy image of the patient at a current moment during a radiation therapy process.

In some embodiments, the above-mentioned fluoroscopy image include at least one fluoroscopy image in at least one angle.

In some embodiments, the fluoroscopy image may be a low-dimensional (such as a two-dimensional plane) kilovolt level (KV) cone beam computerized tomography (CBCT) fluoroscopy image.

For example, the image harvesting device 101 may be a CBCT device, and the CBCT device utilizes a cone beam X-ray source and a two-dimensional detector to scan around the patient and generate a plurality of two-dimensional (2D) plane fluoroscopy images.

In some embodiments, the intelligent agent 102 is configured to acquire a fluoroscopy image of a patient at a current moment during a radiation therapy process and a target image taken for the patient before radiation therapy; and control the radiation therapy process for the patient based on the fluoroscopy image and a target image taken for the patient before radiation therapy.

In one implementation, the intelligent agent 102 is configured to acquire the fluoroscopy image and the target image taken for the patient before radiation therapy in real time, determine spatial position information of a target area of the patient at the current moment based on the fluoroscopy image and the target image taken for the patient before radiation therapy.

In another implementation, the intelligent agent 102 is configured to determine a motion trend and a dose deviation of the target area based on the spatial position information of the target area at the current moment.

Herein, the dose deviation is a difference between a cumulative dose administered actually in vivo and a cumulative dose in vivo that should have been achieved at the current moment as planned.

In yet another implementation, the intelligent agent 102 is configured to determine a radiation therapy control plan for the patient based on the motion trend and the dose deviation of the target area.

It should be noted that the above-mentioned intelligent agent 102 may be a virtual intelligent agent, and the virtual intelligent agent may exist in the form of a computer program product or computer, which relies on algorithms and data to interact with the external environment, and has no physical carrier. However, the virtual intelligent agent may be deployed on an electronic device. The electronic device may be at least one of: a smartphone, a smart watch, a desktop computer, a laptop, a virtual reality terminal, a augmented reality terminal, a wireless terminal, or a laptop-type portable computer; or may also be an independent physical server, or a server cluster or distributed file system composed of multiple physical servers; or may be at least one of cloud servers providing basic cloud computing services such as cloud services, cloud databases, cloud computing, cloud functions, cloud storage, network services, cloud communication, middleware services, domain name services, security services, content delivery networks, and big data or artificial intelligence platforms, which is not limited in the embodiments of the present disclosure.

The above-mentioned intelligent agent 102 may also be a physical intelligent agent. The physical intelligent agent may be combined with a physical carrier to interact with the external environment through hardware such as sensors and actuators, and embedded software.

In some embodiments, as shown in FIG. 1, the intelligent agent 102 includes a sensing intelligent agent 1021, a prediction intelligent agent 1022, a decision-making intelligent agent 1023, and an evolution intelligent agent 1024.

In some embodiments, the sensing intelligent agent 1021 is configured to acquire the fluoroscopy image and the target image taken for the patient before radiation therapy in real time, determine spatial position information of a target area of the patient at the current moment based on the fluoroscopy image and the target image taken for the patient before radiation therapy.

In one implementation, the sensing intelligent agent 1021 is configured to acquire the fluoroscopy image and the target image taken for the patient before radiation therapy in real time; input the fluoroscopy image and the target image taken for the patient before radiation therapy into the sensing intelligent agent, and perform fusion and comparison on the fluoroscopy image and the target image taken for the patient before radiation therapy through the sensing intelligent agent, and infer the spatial position information of the target area of the patient at the current moment based on an image obtained after the fusion and comparison are performed.

In some embodiments, the sensing intelligent agent 1021 sends the spatial position information of the target area at the current moment to the prediction intelligent agent 1022 based on a fixed period.

For example, the sensing intelligent agent 1021 sends the spatial position information of the target area at the current moment to the prediction intelligent agent 1022 thirty times per second.

In some embodiments, the prediction intelligent agent 1022 is configured to determine a motion trend and a dose deviation of the target area based on the spatial position information of the target area at the current moment.

In one implementation, the prediction intelligent agent 1022 determines the motion trend of the target area based on the spatial position information of the target area of the patient at the current moment and spatial position information of the target area of the patient at least one previous moment; and determines, based on the motion trend of the target area in conjunction with a dose deviation at a previous moment and a dose distribution increment predicted for a next moment, an actual dose deviation of the target area at the current moment and predicts a dose deviation for the next moment.

In some embodiments, the prediction intelligent agent 1022 is configured to determine the radiation therapy control plan for the patient based on the motion trend and the dose deviation of the target area.

In some embodiments, the prediction intelligent agent 1022 is further configured to output a confidence level of the motion trend and a confidence level of the dose deviation of the target area determined by the prediction intelligent agent.

In some embodiments, in response to that the confidence level of the motion trend and the confidence level of dose deviation of the target area determined by the prediction intelligent agent 1022 is greater than or equal to a preset threshold, the radiation therapy control plan for the patient is determined based on the motion trend and the dose deviation of the target area.

A specific value of the preset threshold is not limited in the present disclosure, and the preset threshold may be determined according to the actual situations.

In some embodiments, the prediction intelligent agent sends a prediction result (the motion trend and the dose deviation) with a label of a confidence level to the decision-making intelligent agent 1023.

In some embodiments, the decision-making intelligent agent 1023 is configured to determine the radiation therapy control plan for the patient based on the motion trend and the dose deviation of the target area.

In one implementation, after inputting the motion trend and dose deviation of the target area into the decision-making intelligent agent 1023, the decision-making intelligent agent 1023 determines whether radiation therapy is capable of continuing based on the motion trend and the dose deviation of the target area, and in response to that radiation therapy is capable of continuing, performs a first radiation therapy control plan; and in response to that radiation therapy is not capable of continuing, performs a second radiation therapy control plan.

In one implementation, the above-mentioned first radiation therapy control plan is to continue treatment without adjusting a radiation therapy parameter or with fine-tuning the radiation therapy parameter based on the motion trend and the dose deviation.

In one implementation, the above-mentioned second radiation therapy control plan is to control radiation therapy to stop and/or issue a safety alarm to a staff.

For example, the decision-making intelligent agent 1023 sends a control instruction to a treatment device to control the radiation therapy to stop and/or issue a safety alarm to a staff.

In some embodiments, the evolution intelligent agent 1024 is configured to analyze model learning material, and determines a model update scheme for other intelligent agents to be updated except for the evolution intelligent agent 1024.

Herein, a model update scheme is used to indicate whether to update an intelligent agent to be updated and a learning material related to the intelligent agent to be updated.

In some embodiments, the above-mentioned model learning material includes at least one of: images taken in respective treatment sessions, a treatment effect evaluation log, postoperative follow-up data, and a model parameter of another medical center.

In one implementation, the evolution intelligent agent 1024 updates a parameter of an intelligent agent model according to a preset period, for example, the evolution intelligent agent 1024 updates the parameter of the intelligent agent model every day.

Differences between different intelligent agents may be shown in Table 1:

**Table 1**

| Type of Intelligent Agent | Effect | Technology | Interaction Mechanism |
|---|---|---|---|
| Sensing Intelligent Agent | Acquiring and fusing a plurality of kinds of image data in real time | Lightweight feature extraction network + multi head attention fusion | Pushing spatial position information to prediction intelligent agent |
| Prediction Intelligent Agent | Predicting a motion trend and a dose deviation of a target area | Time-Space Separation Transformer + Confidence Level Evaluation Model | Outputting a prediction result with a label of confidence level |
| Decision-Making Intelligent Agent | Generating dynamic adjustment strategies and performing security control | Hierarchical reinforcement learning + interpretable decision tree | Sending a control instruction, pushing alarm information or performing real-time compensation |
| Evolution Intelligent Agent | Managing knowledge base and optimizing system continuously | Online self-learning ability | Updating model parameters and managing core knowledge |

In some embodiments, the above-mentioned sensing intelligent agent 1021, prediction intelligent agent 1022, decision-making intelligent agent 1023, and evolution intelligent agent 1024 may be deployed on the same device, working together under the hardware resources of the same device, or deployed separately on different devices, with communication and collaboration between devices through a network.

The system architecture and service scenarios described in the embodiments of the present disclosure are intended to illustrate the technical solutions of the embodiments of the present disclosure more clearly, and do not constitute a limitation on the technical solutions provided in the embodiments of the present disclosure. A person of ordinary skill in the art may appreciate that with the evolution of network architecture and the emergence of new service scenarios, the technical solutions provided in the embodiments of the present disclosure are also applicable to similar technical problems.

It should be noted that the above-mentioned sensing intelligent agent 1021, prediction intelligent agent 1022, decision-making intelligent agent 1023, and evolution intelligent agent 1024 may all be virtual intelligent agents, where the sensing intelligent agent 1021, the prediction intelligent agent 1022, the decision-making intelligent agent 1023, and the evolution intelligent agent 1024 may be deployed on the same electronic device, or at least one or all of the sensing intelligent agent 1021, the prediction intelligent agent 1022, the decision-making intelligent agent 1023 and the evolution intelligent agent 1024 may be deployed on different electronic devices. In a case where the above-mentioned sensing intelligent agent 1021, prediction intelligent agent 1022, decision-making intelligent agent 1023 and evolution intelligent agent 1024 may all be physical intelligent agents, the sensing intelligent agent 1021, the prediction intelligent agent 1022, the decision-making intelligent agent 1023 and the evolution intelligent agent 1024 are different physical agents. Of course, a part of the above-mentioned sensing intelligent agent 1021, prediction intelligent agent 1022, decision-making intelligent agent 1023 and evolution intelligent agent 1024 c may be virtual intelligent agents, while the remaining agents are physical intelligent agents, which are not limited in the embodiments of the present disclosure.

The radiation therapy control system in the embodiments of the present disclosure is introduced above.

The radiation therapy control method in the embodiments of the present disclosure may be applied to the intelligent agents in the above-mentioned radiation therapy control system. The radiation therapy control method provided in the embodiments of the present disclosure are described below with reference to the accompanying drawings.

As shown in FIG. 2, the radiation therapy control method includes the following steps.

In S100, a fluoroscopy image of a patient taken at a current moment are acquired during a radiation therapy process.

In some embodiments, the above-mentioned fluoroscopy image includes at least one fluoroscopy image in at least one angle, and a fluoroscopy image has advantages such as fast imaging speed and high real-time performance.

For example, the fluoroscopy image may be a kilovolt level Cone Beam Computerized Tomography (KV CBCT) image.

For example, the fluoroscopy image may be a low dimensional X-ray KV fluoroscopy image (two-dimensional image).

In some embodiments, the fluoroscopy image may be a dual KV fluoroscopy image taken at a first angle and a second angle, respectively.

In some embodiments, an angle difference between the first angle and the second angle is greater than or equal to a preset angle.

Herein, the preset angle may be any angle between 70-110 degrees, such as 70 degrees, 80 degrees, 90 degrees, 100 degrees, or 110 degrees.

In S200, the radiation therapy process for the patient is controlled through at least one intelligent agent based on the fluoroscopy image and a target image taken for the patient before radiation therapy.

In some embodiments, the target image includes at least one of: a CT (Computed Tomography) image, an MRI (Magnetic Resonance Imaging) image, or a PET (Positron Emission Tomography) image.

In some embodiments, the target image may be a three-dimensional image, and the target image contains richer information than a single fluoroscopy image.

In some embodiments, the at least one intelligent agent is configured to acquire the fluoroscopy image and the target image taken for the patient before radiation therapy in real time, determine spatial position information of a target area (i.e., a specific area that needs to receive radiation therapy) of the patient at the current moment based on the fluoroscopy image and the target image taken for the patient before radiation therapy.

In some embodiments, the at least one intelligent agent is further configured to determine a motion trend and a dose deviation of the target area based on the spatial position information of the target area at the current moment.

For example, the spatial position information of the target area includes three-dimensional coordinates, a shape, a size, and other data of the target area.

Herein, the dose deviation is a difference between a cumulative dose administered actually in vivo and a cumulative dose in vivo that should have been achieved at the current moment as planned.

In some embodiments, at least one of the aforementioned intelligent agents is further configured to determine the radiation therapy control plan for the patient based on the motion trend and the dose deviation of the target area.

In some embodiments, a time period for controlling the radiation therapy process for the patient is shorter than or equal to a preset time period.

Herein, the above-mentioned preset time period is determined based on a respiratory cycle.

For example, the preset time period may be 1/10 (such as 400 ms) of the respiratory cycle.

It can be understood that, through the at least one intelligent agent, controlling the radiation therapy process is implemented based on the fluoroscopy image taken for the patient in real time during the radiation therapy process and the historical medical images of the patient, thus rich image information may be integrated to accurately acquire changes in the target area of the patient, and capabilities of information processing and decision-making of the intelligent agent are used to dynamically regulate the radiation therapy process, improving the accuracy and safety of radiation therapy, thereby assisting in achieving precise targeting of the target area and enhancing the overall effect of radiation therapy.

In some embodiments, the at least one intelligent agent includes a sensing intelligent agent, and the sensing intelligent agent is configured to capture and fuse multi-source image data in real time.

In some embodiments, the fluoroscopy image and the target image taken for the patient before radiation therapy is acquired through the sensing intelligent agent 1021 in real time, thus to determine spatial position information of a target area of the patient at the current moment based on the fluoroscopy image and the target image taken for the patient before radiation therapy. Specifically, as shown in FIG. 3, it may be implemented as follows.

In S301, the fluoroscopy image and the target image taken for the patient before radiation therapy are acquired in real time.

In some embodiments, at least one fluoroscopy image in at least one angle (such as a dual KV fluoroscopy image) is acquired in real time (i.e., input for sensing). Herein, information contained in a dual KV fluoroscopy image is richer compared to a fluoroscopy image in a single angle.

In S302, the fluoroscopy image and the target image taken for the patient before radiation therapy are input into the sensing intelligent agent, and fusion and comparison are performed on the fluoroscopy image and the target image taken for the patient before radiation therapy through the sensing intelligent agent, and the spatial position information of the target area of the patient at the current moment is inferred based on an image obtained after the fusion and comparison are performed.

In one implementation, processing of fusion and comparison may be performed on an image itself. The fluoroscopy image and the target image taken for the patient before radiation therapy are aligned to the same coordinate system, ensuring that corresponding anatomical structures in these two images are spatially aligned. Subsequently, the aligned two images are superimposed and fused to form a fused image. Multiple regions are then divided on the fused image, centered around the known position of the target area in the target image, to determine its corresponding region in the fused image. Within this region, by calculating similarity metrics (such as mean squared error, mutual information, etc.) of pixel grayscale values, a spatial matching degree between the part of the fluoroscopy image and the part of the target image at the current moment is compared. If the matching degree is below a certain threshold, it indicates that there may be a target area position deviation or other changes. Further analysis of the feature changes in the surrounding regions is conducted to infer a possible deviation direction and distance of the target area, ultimately speculating on the spatial position information of the target area of the patient at the current moment.

In one implementation, processing of fusion and comparison may be performed for features of an image. By utilizing a lightweight feature extraction network, image features are extracted from both the fluoroscopy image and the target image taken for the patient before radiation therapy. For instance, features such as the shape, boundary, and size of the target area in the images are extracted. Through feature concatenation or other more complex fusion strategies (such as a multi-head attention fusion mechanism), the extracted features are fused to obtain a fused image. Subsequently, each voxel (or pixel) and its neighboring features in the fused image are encoded to form a feature vector. These feature vectors are then compared with corresponding feature vectors of the target image to construct a feature difference map. By analyzing the distribution pattern of the difference map, areas with significant changes in features around the target area are identified. Based on this, the spatial position information of the target area at the current moment may be inferred by combining spatial geometric relationships.

In some embodiments, a large amount of image data of the known position of the target area is used to train the sensing intelligent agent. An input of a model is fused image features, and an output is the spatial position information (such as coordinates, shape parameters, etc.) of the target area at the current moment. During the training process, the model learns a mapping relationship between image features and the position of the target area by adjusting internal parameters. When a new fluoroscopy image and target image are input, fused image features are analyzed by a trained model in the sensing intelligent agent to predict the spatial position information of the target area at the current moment. For example, the model may distinguish position changes of the target area caused by factors such as breathing in the fluoroscopy image based on learned feature patterns, and convert them into position representations corresponding to a coordinate system for the target image.

It can be understood that the sensing intelligent agent is capable of improving the accuracy of radiation therapy positioning by integrating a plurality of kinds of image data, and its real-time processing capability may monitor the spatial position changes of the target area, providing more accurate and effective information for radiation therapy control.

In some embodiments, the at least one intelligent agent includes a prediction intelligent agent, and the prediction intelligent agent is configured to predict a motion trend and a dose deviation for an organ.

In some embodiments, a motion trend and a dose deviation of the target area are determined through the prediction intelligent agent based on the spatial position information of the target area at the current moment. Specifically, the spatial position information of the target area of the patient at the current moment is input into the prediction intelligent agent. The prediction intelligent agent determines the motion trend of the target area based on the spatial position information of the target area of the patient at the current moment and spatial position information of the target area of the patient at a previous moment, and determines the dose deviation of the target area based on the motion trend of the target area.

In one implementation, the prediction intelligent agent utilizes a trained time-space separation transformer model to process the spatial position information of the target area of the patient at least one previous moment, and determine the motion trend of the target area; and determines, based on the motion trend of the target area in conjunction with a dose deviation at a previous moment and a dose distribution increment predicted for a next moment, an actual dose deviation of the target area at the current moment and predicts a dose deviation for the next moment.

For example, features are extracted from the spatial position information of the target area at the current moment and at least one previous moment, and these features may include changes in position coordinates, shape change parameters, volume change rate, etc., for the target area. For example, the changes in position coordinates may be obtained by calculating a difference between center coordinates of the target area at the current moment and at least one previous moment. Then, using the trained time-space separation transformer model, the motion trend of the target area may be predicted based on the spatial position information of the target area at the current moment and at least one previous moment. The motion trend may be expressed as a possible displacement direction, velocity, acceleration and other parameters for the target area in the future period of time. Furthermore, based on the predicted motion trend of the target area, a finite element analysis method or a dose calculation model are used to predict the dose distribution increment at the next moment. Finally, the dose deviation of the target area at the next moment is predicted by integrating the dose deviation at the current moment and the dose distribution increment at the next moment (considering a cumulative effect and dynamic change law of dose).

In some embodiments, the prediction intelligent agent is further configured to output a confidence level of the motion trend and a confidence level of the dose deviation of the target area determined by the prediction intelligent agent.

In one implementation, the prediction intelligent agent determines a confidence level of the motion trend and a confidence level of the dose deviation of the target area through a confidence evaluation model.

In some embodiments, to ensure the reliability of prediction, a threshold for confidence level may be set, and a radiation therapy control plan for the patient may be determined in a case where the confidence level of the motion trend and the confidence level of the dose deviation of the target area meet a threshold.

Correspondingly, based on the motion trend and dose deviation of the target area, determining the radiation therapy control plan for the patient may be specifically implemented as: in response to that the confidence level of the motion trend and the confidence level of dose deviation of the target area determined by the prediction intelligent agent is greater than or equal to a preset threshold, determining the radiation therapy control plan for the patient based on the motion trend and the dose deviation of the target area.

In some embodiments, the at least one intelligent agent includes a decision-making intelligent agent, and the decision-making intelligent agent is configured to generate dynamic adjustment measurements and perform security control during the radiation therapy process.

In some embodiments, the radiation therapy control plan for the patient is determined through the decision-making intelligent agent based on the motion trend and the dose deviation of the target area. Specifically, the motion trend and the dose deviation of the target area are input into the decision-making intelligent agent, and whether radiation therapy is capable of continuing is determined based on the motion trend and the dose deviation of the target area, and in response to that radiation therapy is capable of continuing, a first radiation therapy control plan is performed, and in response to that radiation therapy is not capable of continuing, a second radiation therapy control plan is performed.

In some embodiments, the first radiation therapy control plan is to continue treatment without adjusting a radiation therapy parameter or with fine-tuning the radiation therapy parameter based on the motion trend and the dose deviation.

In some embodiments, the second radiation therapy control plan is to control radiation therapy to stop and/or issue a safety alarm to a staff.

In one implementation, in a case where the motion trend and dose deviation of the target area are small, it indicates that the motion trend and dose deviation of the target area are in a normal state. At this time, it is determined that radiation therapy is capable of continuing and the first radiation therapy control plan may be performed.

In one implementation, in a case where the motion trend and dose deviation of the target area are large, it indicates that the current motion trend and dose deviation of the target area may be in an abnormal state. At this time, it is determined that radiation therapy is not capable of continuing, and the second radiation therapy control plan is then performed.

In some embodiments, the decision-making intelligent agent dynamically adjusts the radiation therapy control plan in conjunction with a hierarchical reinforcement learning mechanism and a interpretable decision tree mechanism.

It can be understood that the decision-making intelligent agent identifies the normal state and abnormal state based on the motion trend and dose deviation of the target area, and performs corresponding control plans. On the one hand, it can ensure the continuity of radiation therapy and improve treatment efficiency. On the other hand, it can also ensure patient safety and facilitate abnormal diagnosis and treatment when abnormalities occur.

In some embodiments, the at least one intelligent agent further includes an the evolution intelligent agent, and the evolution intelligent agent is configured to manage a knowledge base related to radiation therapy data and continuously optimize the radiation therapy control system.

As shown in FIG. 4, the radiation therapy control method provided in the embodiments of the present disclosure further includes the following steps.

In S401, a model learning material is acquired.

Herein, the model learning material provides data basis for updating an intelligent agent.

In some embodiments, the above-mentioned model learning material includes at least one of: images taken in respective treatment sessions, a treatment effect evaluation log, postoperative follow-up data, and a model parameter of another medical center.

In one implementation, the model learning material may be stored in a dedicated database, and the evolution intelligent agent may interface with the database through a dedicated interface to acquire or read the model learning material from the database.

In S402, the model learning material is input into the evolution intelligent agent, the model learning material is analyzed through the evolution intelligent agent, and a model update scheme for another intelligent agent to be updated in the at least one intelligent agent except for the evolution intelligent agent is determined.

Herein, a model update scheme is used to indicate whether to update an intelligent agent to be updated and a learning material related to the intelligent agent to be updated.

In some embodiments, due to differences in model architecture, algorithm principles, and task targets among different intelligent agents, the evolution intelligent agent needs to combine model characteristics of different intelligent agents to determine model update schemes for them. A model update scheme may include adjusting hyperparameters of a model (such as a learning rate, a regularization coefficient, etc.), modifying a model structure (such as increasing or decreasing a number of network layers, changing a connection manner between layers, etc.), updating a training dataset of a model (such as adding new sample categories, correcting mislabeled data, etc.), or introducing new algorithm components, etc.

In some embodiments, due to the collaborative working relationship between intelligent agents, the evolution intelligent agent further needs to consider compatibility and collaborative effects between intelligent agents when determining model update schemes. For example, in an intelligent agent team, one intelligent agent is responsible for feature extraction, while another intelligent agent is responsible for making decisions based on these features. When updating the model used for feature extraction, it is important to ensure that it does not have a negative impact on the input of the intelligent agent for decision-making. At the same time, it may also be necessary to make appropriate adjustments to the model used for decision-making to adapt to the new feature representation.

In one implementation, the evolution intelligent agent analyzes the model learning material, determines an intelligent agent to be updated, and sends an update instruction to the intelligent agent to be updated. The intelligent agent to be updated then obtains relevant data by itself based on the update instruction, and performs updating based on the relevant data.

In one implementation, the evolution intelligent agent analyzes the model learning material, determines an intelligent agent to be updated, and sends an update instruction and relevant data (such as the model learning material, etc.) to the intelligent agent to be updated, thus to make the intelligent agent to be updated to perform model update and optimization according to the received relevant data.

For example, after each treatment session is completed, the evolution intelligent agent indicates personalized models in different intelligent agents to perform update and optimization according to images taken in this treatment session (including clear update instructions and corresponding training samples), to enable the models to adjust their own parameters based on the latest image data and improve precise adaptation to the patient's treatment process.

For example, a doctor provide professional evaluations for the treatment effectiveness of the model based on a real-time treatment result. After the evolution intelligent agent acquires these professional evaluation data, it accurately determines the relevant data for each intelligent agent model. Based on this data, the evolution intelligent agent then determines whether the respective intelligent agent models need to be updated. If it is determined that an update is needed, relevant data corresponding to the respective intelligent agent models may be sent in a targeted manner, so that the respective intelligent agent models may perform update and optimization based on the received data, ensuring that the models meet the actual clinical treatment needs.

For example, a doctor conduct a comprehensive and systematic evaluation for the treatment effectiveness of intelligent agent models based on follow-up data after treatment. The evolution intelligent agent accurately extracts relevant data from the evaluation information of the respective models, and through in-depth analysis of these data, determines whether the respective intelligent agent models need to be updated. In a case of determining to perform update, corresponding relevant data to the respective intelligent agent models is sent, so that the respective intelligent agent models may perform update and optimization based on the obtained data, improve model accuracy, and thereby enhance the treatment effect for the patient.

For example, the evolution intelligent agent interfaces with a federated learning platform to obtain model parameters of other medical centers. Subsequently, the parameters of other medical center corresponding models are sent to the respective intelligent agent models separately. Through cross comparison and fusion analysis of the model parameters of a plurality of medical centers, the parameters of the respective intelligent agent models are updated to achieve cross center and multi-dimensional model optimization and improvement, enhancing the universality and accuracy of the models.

The radiation therapy control method provided in the present disclosure may be introduced through a complete embodiment.

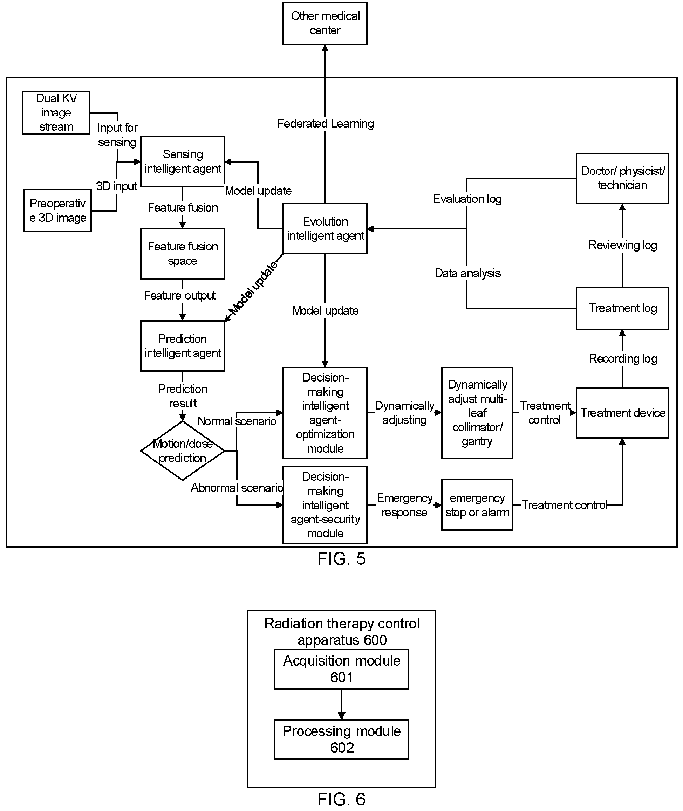

As shown in FIG. 5, a dual KV image stream and a preoperative 3D image are input as original data into the sensing intelligent agent. The sensing intelligent agent receives these data, extracts feature information in the data, and delivers processed feature information to a feature fusion space for fusion, forming a comprehensive feature representation. The fused features are then output to the prediction intelligent agent.

Based on the received feature information, the prediction intelligent agent uses a pre-trained model to predict a motion and a dose distribution of the patient. A prediction result include a motion trend and a dose deviation, and whether it is currently an abnormal scenario or in a normal scenario is determined based on the prediction result.

In a case of determining that it is a normal scenario, an optimization module of the decision-making intelligent agent dynamically adjusts parameters of a multi-leaf collimator (MLC)/gantry based on a prediction result of the prediction intelligent agent, in order to optimize an irradiation path and a dose distribution of radiation beams, so as to focus the radiation beams more accurately on the tumor site and reduce a irradiation dose on surrounding normal tissues. The adjusted parameters are sent to a treatment device, and the treatment device performs corresponding treatment control operations and records treatment logs. In a case of determining that it is an abnormal scenario, a security module of the decision-making intelligent agent initiates an emergency response mechanism, sends an emergency stop or alarm signal to the treatment device, causing the device to immediately stop treatment or issue an alarm to alert a medical staff, in order to ensure the safety of the patient.

Next, the evolution intelligent agent receives evaluations made by doctors, physicists, or technicians after reviewing the treatment logs, and collects key data from the treatment logs. Based on these feedback data, the evolution intelligent agent updates and optimizes the models of the sensing intelligent agent, prediction intelligent agent, and decision-making intelligent agent in the entire system.

In addition, as shown in FIG. 5, the evolution intelligent agent also collaborates with other medical centers through federated learning. On the premise of protecting patient privacy, encrypting the model update information of this center and sharing it with other centers, while receiving update information from other centers, integrating data and experience from all parties, further enhancing the adaptability and generalization ability of the entire system in different medical scenarios.

The solutions provided in the embodiments of the present disclosure are introduced above from the perspective of methods. In order to implement the above functions, the radiation therapy control apparatus includes corresponding hardware structures and/or software modules for implementing various functions. Those skilled in the art should easily realize that the present disclosure can be implemented in the form of hardware or a combination of hardware and computer software in combination with units and algorithm steps described in the embodiments of the description. Whether a certain function is implemented by hardware or by computer software driving hardware depends on the specific application and design constraint conditions of the technical solutions. A professional technician may use different methods to implement the described functions for each specific application, but such implementation should not be considered beyond the scope of the present disclosure.

The embodiments of the present disclosure may divide the radiation therapy control apparatus into functional modules exemplarily according to the above methods. For example, the radiation therapy control apparatus may include various functional modules divided corresponding to various functions, or two or more functions may be integrated into one processing module. The above-mentioned integrated module may be implemented in the form of hardware, or may be implemented in the form of software functional module. It should be noted that the division of the modules in the embodiments of the present disclosure is illustrated, which is only a logical functional division, and there may be other division manners in an actual implementation.

FIG. 6 is a schematic diagram of structures of a radiation therapy control apparatus provided in the embodiments of the present disclosure. Referring to FIG. 6, the radiation therapy control apparatus 600 includes an acquisition module 601 and a processing module 602.

The acquisition module 601 is configured acquire a fluoroscopy image of a patient taken at a current moment during a radiation therapy process;

the processing module 602 is configured to control the radiation therapy process for the patient through at least one intelligent agent based on the fluoroscopy image and a target image taken for the patient before radiation therapy.

In some embodiments, the at least one intelligent agent is configured to perform the following operations: acquiring the fluoroscopy image and the target image taken for the patient before radiation therapy in real time, determining spatial position information of a target area of the patient at the current moment based on the fluoroscopy image and the target image taken for the patient before radiation therapy; determining a motion trend and a dose deviation of the target area based on the spatial position information of the target area at the current moment, where the dose deviation is a difference between a cumulative dose administered actually in vivo and a cumulative dose in vivo that should have been achieved at the current moment as planned; determining a radiation therapy control plan for the patient based on the motion trend and the dose deviation of the target area.

In some embodiments, the at least one intelligent agent includes a sensing intelligent agent; acquiring the fluoroscopy image and the target image taken for the patient before radiation therapy in real time, and determining the spatial position information of the target area of the patient at the current moment based on the fluoroscopy image and the target image taken for the patient before radiation therapy include: acquiring the fluoroscopy image and the target image taken for the patient before radiation therapy in real time; inputting the fluoroscopy image and the target image taken for the patient before radiation therapy into the sensing intelligent agent, and performing fusion and comparison on the fluoroscopy image and the target image taken for the patient before radiation therapy through the sensing intelligent agent, and inferring the spatial position information of the target area of the patient at the current moment based on an image obtained after the fusion and comparison are performed.

In some embodiments, the at least one intelligent agent further includes a prediction intelligent agent; determining the motion trend and the dose deviation of the target area based on the spatial position information of the target area at the current moment includes: inputting the spatial position information of the target area of the patient at the current moment into the prediction intelligent agent, and determining the motion trend of the target area based on the spatial position information of the target area of the patient at the current moment and spatial position information of the target area of the patient at least one previous moment through the prediction intelligent agent; and determining, based on the motion trend of the target area in conjunction with a dose deviation at a previous moment and a dose distribution increment predicted for a next moment, an actual dose deviation of the target area at the current moment and predicting a dose deviation for the next moment.

In some embodiments, the prediction intelligent agent is further configured to output a confidence level of the motion trend and a confidence level of the dose deviation of the target area determined by the prediction intelligent agent; determining the radiation therapy control plan for the patient based on the motion trend and the dose deviation of the target area includes: in response to that the confidence level of the motion trend and the confidence level of dose deviation of the target area determined by the prediction intelligent agent is greater than or equal to a preset threshold, determining the radiation therapy control plan for the patient based on the motion trend and the dose deviation of the target area.

In some embodiments, the at least one intelligent agent further includes a decision-making intelligent agent; determining the radiation therapy control plan for the patient based on the motion trend and the dose deviation of the target area includes: inputting the motion trend and the dose deviation of the target area into the decision-making intelligent agent, and determining whether radiation therapy is capable of continuing based on the motion trend and the dose deviation of the target area, and in response to that radiation therapy is capable of continuing, performing a first radiation therapy control plan, and in response to that radiation therapy is not capable of continuing, performing a second radiation therapy control plan.

In some embodiments, the first radiation therapy control plan is to continue treatment without adjusting a radiation therapy parameter or with fine-tuning the radiation therapy parameter based on the motion trend and the dose deviation; the second radiation therapy control plan is to control radiation therapy to stop and/or issue a safety alarm to a staff.

In some embodiments, the at least one intelligent agent further includes an evolution intelligent agent; the acquisition module 601 is further configured to acquire a model learning material; and the processing module 602 is further configured to: input the model learning material into the evolution intelligent agent, analyze the model learning material through the evolution intelligent agent, and determine a model update scheme for another intelligent agent to be updated in the at least one intelligent agent except for the evolution intelligent agent, where a model update scheme is used to indicate whether to update an intelligent agent to be updated and a learning material related to the intelligent agent to be updated.

In some embodiments, the model learning material includes at least one of: images taken in respective treatment sessions, a treatment effect evaluation log, postoperative follow-up data, and a model parameter of another medical center.

In some embodiments, the fluoroscopy image includes at least one fluoroscopy image in at least one angle.

In some embodiments, the target image includes at least one of: a CT image, an MRI image, or a PET image.

In some embodiments, a time period for controlling the radiation therapy process for the patient through the at least one intelligent agent based on the fluoroscopy image and the target image taken for the patient before radiation therapy is shorter than or equal to a preset time period, where the preset time period is determined based on a respiratory cycle.

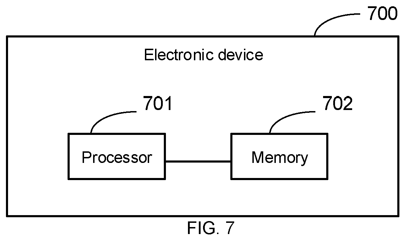

FIG. 7 is a schematic diagram of structures of an electronic device provided in the embodiments of the present disclosure. As shown in FIG. 7, the electronic device 700 includes, but not limited to: a processor 701 and a memory 702.

Herein, the above-mentioned memory 702 is configured to store instructions that are executable for the above-mentioned processor 701. It can be understood that the above-mentioned processor 701 is configured to execute instructions, so as to implement the radiation therapy control method in the above-mentioned embodiments.

It should be noted that those skilled in the art may understand that structures of the electronic device shown in FIG. 7 do not constitute a limitation on the electronic device. The electronic device may include more or fewer components than those shown in FIG. 7, or certain components may be combined, or components may be arranged in different ways.

The processor 701 is a control center of the electronic devices, which connects various parts of the entire electronic device through various interfaces and circuits. By running or executing software programs and/or modules stored in the memory 702, and calling data stored in the memory 702, the processor 701 may perform various functions of the electronic device and process data, thereby monitoring the electronic device as a whole. The processor 701 may include one or more processing units. Optionally, the processor 701 may integrate an application processor and a modulation and demodulation processor, where the application processor mainly handles operating systems, user interfaces, and application programs, and the modulation and demodulation processor mainly handles wireless communication. It can be understood that the above-mentioned modulation and demodulation processor may not be integrated into the processor 701.

The memory 702 may be configured to store software programs and various data. The memory 702 may mainly include an area for storing programs and an area for storing data, where the area for storing programs may store application programs required by an operating system, or at least one functional module (such as a determination unit, a processing unit, etc.), etc. In addition, the memory 702 may include a high-speed random access memory, and may further include a non-volatile memory (e.g., at least one disk storage component, flash memory component, or other volatile solid-state storage components).

In an exemplary embodiment, a computer-readable storage medium including instructions is further provided, such as the memory 702 including the instructions, and the above-mentioned instructions may be executed by the processor 701 of the electronic device 700, so as to implement the methods described in the above-mentioned embodiments.

In practical implementation, both the acquisition module 601 and the processing module 602 in FIG. 6 may be implemented by the processor 701 in FIG. 7 by calling the computer programs stored in the memory 702. The specific execution process may refer to the description of method sections in the above-mentioned embodiments, which will not be repeated herein.

Optionally, the computer-readable storage medium may be a non-transitory computer-readable storage medium, for example, the non-transitory computer-readable storage medium may be a read-only memory (ROM), a random access memory (RAM), a CD-ROM, a magnetic tape, a floppy disk, an optical data storage device, etc.

In an exemplary embodiment, a computer program product including one or more instructions is further provided in the embodiments of the present disclosure, and the one or more instructions ma be executed by the processor 701 of the electronic device, so as to implement the methods described in the above-mentioned embodiments.

It should be noted that when the instructions in the computer-readable storage medium or one or more instructions in the computer program product are executed by the processor of the electronic device, the various processes of the above method embodiments may be implemented, and the same technical effects as the above methods may be achieved, which will not be repeated herein to avoid repetition.

Through the description of the above implementations, those of ordinary skill in the art may clearly understand that for the convenience and simplicity of the description, the division of the above functional modules is given merely as an example. In practical applications, the above functions can be allocated to different functional modules according to requirements, that is, the internal structure of the apparatus may be divided into different functional modules to complete all or part of the functions described above.

In the several embodiments provided in the present disclosure, it should be understood that the disclosed apparatuses and methods may be implemented in other ways. For example, the apparatus embodiments described above are only illustrative. For example, the division of modules or units is only a logical functional division. In actual implementation, there may be other division methods, for example, multiple units or components can be combined or integrated into another apparatus, or some features can be ignored or not performed. On the other hand, the coupling or direct coupling or communicative connection with each other as shown or discussed may be indirect coupling or communicative connection of apparatus or units via some interfaces, which may be electrical, mechanical, or in other forms.

The units described as separate components may or may not be physically separated, and the component(s) shown as units may be one physical unit or multiple physical units, that is, may be located in one place, or may be distributed to multiple different places. A part or all of the units may be selected according to actual needs, to implement the purpose of the solutions of the embodiments.

In addition, the functional units in the embodiments of the present disclosure may be integrated into a single processing unit or the functional units may exist physically and separately, or two or more units may be integrated into a unit. The above integrated unit may be implemented in the form of hardware, or may be implemented in the form of a software functional unit.

If the integrated unit is implemented in the form of the software functional unit and sold or used as an independent product, the integrated unit may be stored in a readable storage medium. Based on this understanding, the technical solutions of the present disclosure may be essentially, or a part of the technical solutions of the present disclosure that contributes to the prior art may be, all or part of the technical solutions may be, embodied in the form of a software product, and the software product is stored in a storage medium, which includes several instructions to enable a device (which may be a single-chip microcomputer, a chip, etc.) or a processor to perform all or part of steps of methods in various embodiments of the present disclosure. The aforementioned storage media include: a U disk, a mobile hard disk, a ROM, a RAM, a magnetic disk or an optical disk and other media that may store program codes.

In the description of the embodiments of the present disclosure, specific features, structures, materials, or characteristics may be combined in any one or more embodiments or examples in a suitable manner.

The above description is only a specific implementation of the embodiments of the present disclosure, but the protection scope of the embodiments of the present disclosure is not limited thereto, and any skilled in the art familiar with this technical field may easily think of changes or substitutions within the technical scope disclosed in the embodiments of the present disclosure, which should be all covered within the protection scope of the embodiments of the present disclosure. Therefore, the protection scope of the present application be subject to the protection scope of the claims.

## Claims

1. A radiation therapy control method, **characterized in that** the method comprises:
acquiring (S100) a fluoroscopy image of a patient taken at a current moment during a radiation therapy process; and
controlling (S200) the radiation therapy process for the patient through at least one intelligent agent based on the fluoroscopy image and a target image taken for the patient before radiation therapy.

2. The method according to claim 1, wherein the at least one intelligent agent is configured to perform the following operations:
acquiring the fluoroscopy image and the target image taken for the patient before radiation therapy in real time, determining spatial position information of a target area of the patient at the current moment based on the fluoroscopy image and the target image taken for the patient before radiation therapy;
determining a motion trend and a dose deviation of the target area based on the spatial position information of the target area at the current moment, wherein the dose deviation is a difference between a cumulative dose administered actually in vivo and a cumulative dose in vivo that should have been achieved at the current moment as planned;
determining a radiation therapy control plan for the patient based on the motion trend and the dose deviation of the target area.

3. The method according to claim 2, wherein the at least one intelligent agent comprises a sensing intelligent agent (1021); acquiring the fluoroscopy image and the target image taken for the patient before radiation therapy in real time, and determining the spatial position information of the target area of the patient at the current moment based on the fluoroscopy image and the target image taken for the patient before radiation therapy comprise:
acquiring (S301) the fluoroscopy image and the target image taken for the patient before radiation therapy in real time; inputting (S302) the fluoroscopy image and the target image taken for the patient before radiation therapy into the sensing intelligent agent (1021), and performing fusion and comparison on the fluoroscopy image and the target image taken for the patient before radiation therapy through the sensing intelligent agent (1021), and inferring the spatial position information of the target area of the patient at the current moment based on an image obtained after the fusion and comparison are performed.

4. The method according to claim 2, wherein the at least one intelligent agent further comprises a prediction intelligent agent (1022); determining the motion trend and the dose deviation of the target area based on the spatial position information of the target area at the current moment comprises:
inputting the spatial position information of the target area of the patient at the current moment into the prediction intelligent agent (1022), and determining the motion trend of the target area based on the spatial position information of the target area of the patient at the current moment and spatial position information of the target area of the patient at least one previous moment through the prediction intelligent agent (1022); and determining, based on the motion trend of the target area in conjunction with a dose deviation at a previous moment and a dose distribution increment predicted for a next moment, an actual dose deviation of the target area at the current moment and predicting a dose deviation for the next moment.

5. The method according to claim 4, wherein the prediction intelligent agent (1022) is further configured to output a confidence level of the motion trend and a confidence level of the dose deviation of the target area determined by the prediction intelligent agent (1022);
determining the radiation therapy control plan for the patient based on the motion trend and the dose deviation of the target area comprises:
in response to that the confidence level of the motion trend and the confidence level of dose deviation of the target area determined by the prediction intelligent agent (1022) is greater than or equal to a preset threshold, determining the radiation therapy control plan for the patient based on the motion trend and the dose deviation of the target area.

6. The method according to claim 2, wherein the at least one intelligent agent further comprises a decision-making intelligent agent (1023); determining the radiation therapy control plan for the patient based on the motion trend and the dose deviation of the target area comprises:
inputting the motion trend and the dose deviation of the target area into the decision-making intelligent agent (1023), and determining whether radiation therapy is capable of continuing based on the motion trend and the dose deviation of the target area, and in response to that radiation therapy is capable of continuing, performing a first radiation therapy control plan, and in response to that radiation therapy is not capable of continuing, performing a second radiation therapy control plan.

7. The method according to claim 6, wherein the first radiation therapy control plan is to continue treatment without adjusting a radiation therapy parameter or with fine-tuning the radiation therapy parameter based on the motion trend and the dose deviation; the second radiation therapy control plan is to control radiation therapy to stop and/or issue a safety alarm to a staff.

8. The method according to any one of claims 3 to 6, wherein the at least one intelligent agent further comprises an evolution intelligent agent (1024); the method further comprises:
acquiring (S401) a model learning material;
inputting (S402) the model learning material into the evolution intelligent agent (1024), analyzing the model learning material through the evolution intelligent agent (1024), and determining a model update scheme for another intelligent agent to be updated in the at least one intelligent agent except for the evolution intelligent agent (1024), wherein a model update scheme is used to indicate whether to update an intelligent agent to be updated and a learning material related to the intelligent agent to be updated.

9. The method according to claim 8, wherein the model learning material comprises at least one of:
images taken in respective treatment sessions, a treatment effect evaluation log, postoperative follow-up data, and a model parameter of another medical center.

10. The method according to claim 1, wherein the fluoroscopy image comprises at least one fluoroscopy image in at least one angle.

11. The method according to claim 1, wherein the target image comprises at least one of: a computed tomography, CT, image, a magnetic resonance imaging, MRI, image, or a positron emission tomography, PET, image.

12. The method according to claim 1, wherein a time period for controlling the radiation therapy process for the patient through the at least one intelligent agent based on the fluoroscopy image and the target image taken for the patient before radiation therapy is shorter than or equal to a preset time period, wherein the preset time period is determined based on a respiratory cycle.

13. An electronic device (700), **characterized in that** the electronic device (700) comprises:
a processor (701); and
a memory (702) configured to store instructions executable for the processor (701);
wherein the processor (701) is configured to execute the instructions, to implement the method according to any one of claims 1 to 12.

14. A computer-readable storage medium, **characterized in that** computer instructions is stored on the computer-readable storage medium, the computer instructions, upon being run on a computer, cause the computer to perform the method according to any one of claims 1 to 12.

15. A computer program product, **characterized in that** the computer program product comprises computer instructions that, upon being run on an electronic device, cause the electronic device to perform the method according to any one of claims 1 to 12.
